# EUROPÄISCHE PATENTANMELDUNG

(11) **EP 2 570 142 A1**
(43) Veröffentlichungstag der Anmeldung: **20.03.2013**
(21) Anmeldenummer: 12180768.9
(22) Anmeldetag: 17.08.2012
(51) Int. Cl.: A61M 1/10, A61N 1/362, A61N 1/368, A61N 1/365, A61N 1/39, A61M 1/12, A61N 1/37

(54) **Implantierbares kardiales Therapiegerät**

(30) Priorität: 14.09.2011 US 201161534939 P
(71) Anmelder: BIOTRONIK SE & Co. KG, 12359 Berlin (DE)
(72) Erfinder: Dörr, Thomas, 12437 Berlin (DE); Weiss, Ingo, 12435 Berlin (DE)
(74) Vertreter: Lindner-Vogt, Karin L.

(57) **Zusammenfassung**

Die Erfindung betrifft ein implantierbares kardiales Therapiegerät mit einer Herzunterstützungspumpe, einer Stimulationseinheit und einer Steuereinheit. Die Herzunterstützungspumpe ist mit einem Ventrikel eines Herzens und einer zugehörigen Arterie zu verbinden und ausgebildet, im Einsatzfall Blut aus einem jeweiligen Ventrikel in eine jeweils zugehörige Arterie zu pumpen und so den jeweiligen Ventrikel zu unterstützen. Die Stimulationseinheit ist zur elektrischen Stimulation einer Herzkontraktion ausgebildet und die Steuereinheit ist ausgebildet, die Herzunterstützungspumpe und die Stimulationseinheit derart koordiniert anzusteuern, dass die Stimulationseinheit einen synchronisierten Bewegungsablauf der kardialen Kontraktion bewirkt, während die Herzunterstützungspumpe ventrikelunterstützend arbeitet.

## Beschreibung

Die Erfindung betrifft ein implantierbares kardiales Therapiegerät.

Bekannte implantierbare kardiale Therapiegeräte sind z.B. Herzunterstützungspumpe, die mit einem Ventrikel eines Herzens und einer zugehörigen Arterie zu verbinden und ausgebildet ist, im Einsatzfall Blut aus einem jeweiligen Ventrikel in eine jeweils zugehörige Arterie zu pumpen und so den jeweiligen Ventrikel zu unterstützen/entlasten. Insbesondere werden zur Unterstützung einer sehr stark eingeschränkten ventrikulären Pumpfunktion bereits Unterstützungspumpen (VAD) implantiert. Ebenso ist bekannt, dass auch biventrikuläre VAD-Pumpen eingesetzt werden, um Patienten mit Rechts- und Linksherzinsuffizienz adäquat zu behandeln.

Andere bekannte implantierbare kardiale Therapiegeräte sind Herzstimulatoren wie beispielsweise Herzschrittmacher oder Defibrillatoren/Kardioverter mit einer Stimulationseinheit zur elektrischen Stimulation einer Herzkontraktion. Insbesondere werden zur kardialen Resynchronisationstherapie (CRT) bereits biventrikuläre Stimulatoren implantiert, die elektrische Stimulationsimpulse sowohl an einen rechten Ventrikel eines Herzens als auch an einen linken Ventrikel eines Herzens abgeben können, um jeweils eine Kontraktion des jeweiligen Ventrikels zu stimulieren. Im Rahmen einer kardialen Resynchronisationstherapie werden die Stimulationsimpulse so abgegeben, dass die Kontraktionen beider Ventrikel ähnlich wie beim gesunden Herzen miteinander koordiniert sind.

In US 2006/0036127 A1 [0042] ist eine Kombination einer VAD-Pumpe mit einem Schrittmacher beschrieben.

Der Erfindung liegt die Aufgabe zugrunde, ein kardiales Therapiegerät zu schaffen, das neue Therapiemöglichkeiten eröffnet.

Erfindungsgemäß wird ein Kombinationstherapiegerät zur Förderung der Rückbildung bei Patienten mit sehr stark eingeschränkter ventrikulärer Pumpfunktion vorgeschlagen, das eine Kombination aus einer Herzunterstützungspumpe ( auch ventrikulären Assistenzpumpe oder ventricular assist device (VAD) genannt) und einem Herzstimulator mit einer Stimulationseinheit darstellt und eine Steuereinheit aufweist, die mit der Herzunterstützungspumpe und der Stimulationseinheit steuernd verbunden und ausgebildet ist, die Herzunterstützungspumpe und die Stimulationseinheit derart koordiniert anzusteuern, dass die Stimulationseinheit einen synchronisierten Bewegungsablauf der kardialen Kontraktion bewirkt, während die Herzunterstützungspumpe ventrikelunterstützend arbeitet.

Die Steuereinheit wirkt somit als Therapiekoordinationseinheit, die die Pumpfunktion und die Stimulation derart koordiniert, dass neben der ventrikulären Pumpunterstützung ein synchronisierter Bewegungsablauf der kardialen Kontraktion erreicht wird und stellt auf diese Weise einen Zusammenhang zwischen der VAD-Pumpfunktion zur myokardialen "Druck- und Volumenentlastung" und der elektrisch modulierten Kontraktionsdynamik her. Prinzip der Kombinationstherapie ist eine Optimierung der Kontraktionsabläufe und speziell der Klappenschlussmechanismen, um so ein Reverse-Remodeling unterstützen zu können. Zusätzlich kann der VAD-Fluss reduziert werden.

Das erfindungsgemäße implantierbare kardiale Therapiegerät hat den Effekt, ein Reverse-Remodeling - also eine Rückbildung der Krankheit - bei Patienten mit stark eingeschränkter ventrikulärer Pumpfunktion zu verbessern. Das erfindungsgemäße implantierbare kardiale Therapiegerät kann wenigstens in einigen Fällen die Therapieeffizienz ventrikulärer Unterstützungspumpen derart steigern, dass das erreichbare Reverse-Remodeling ausreichend ist, die kardiale Funktion auch ohne VAD-System zu stabilisieren.

Die Erfindung schließt die Erkenntnis ein, dass derzeit bereits ventrikuläre Unterstützungspumpen (VAD) für Patienten mit sehr stark eingeschränkter ventrikulärer Pumpfunktion und myokardialem Perfusionsdefizit erfolgreich eingesetzt werden und dass in einigen wenigen Fällen dabei ein derart erfolgreiches Reverse-Remodeling beobachtet wurde, dass diese Patienten nach einer VAD-Therapiephase dauerhaft ohne ein solches VAD-System weiterleben können.

Allerdings sind dies nur wenige Einzelfälle und es fehlt bisher ein Konzept, die Patienten in der Mehrzahl so erfolgreich zu therapieren, dass der Einsatz der mit vielen Nachteilen verbundenen VAD-Systeme nur für einen relativ kurzen Zeitraum notwendig ist. Damit wäre auch eine kostengünstigere VAD-Therapie möglich.

Die Erfindung schließt außerdem die Erkenntnis ein, dass derzeitige VAD-Systeme bevorzugt druckgesteuert sind und es ein Nachteil dieser Steuerung ist dabei, dass die kardiale Resynchronisation und z.T. auch AV-Synchronisation des Herzzyklus nicht optimiert werden können. Damit gehen diesen Patienten die therapeutischen Benefits einer CRT-Therapie verloren und schränken potentiell das Reverse-Remodeling ein.

Gleiches gilt für die biventrikulären VAD-Systeme, die zwar beide Ventrikel "entlasten" und damit einer Verbesserung der myokardialen Sauerstoffversorgung zuträglich sind, die aber vorhandene Asymmetrie der Kontraktion nicht beseitigen können.

Ein weiterer von den Erfindern erkannter Nachteil linksventrikulärer VAD-Systeme besteht bei zusätzlicher Rechtsherzinsuffizient darin, dass zwar das linksventrikuläre Schlagvolumen gesteigert wird, gleichzeitig aber durch den venösen Rückstrom die Volumenbelastung des rechten Ventrikels zunimmt und damit ein Rechtsherzversagen gefördert wird.

Die Steuereinheit verwirklicht neben einer Therapiekoordinationseinheit, die die Pumpfunktion und die Stimulation derart koordiniert, dass neben der ventrikulären Pumpunterstützung ein synchronisierter Bewegungsablauf der kardialen Kontraktion erreicht wird auch eine Kontraktionsablaufkontrolleinheit, die die Abgabe und ggf. Inhibierung von Stimulationsimpulsen über die Stimulationseinheit oder auch mehrere Stimulationseinheiten steuert.

Vorzugsweise ist die Steuereinheit in Kombination mit der Stimulationseinheit oder mehreren Stimulationseinheiten dazu ausgebildet, die Abgabe von Stimulationsimpulsen für eine biventrikuläre Stimulationstherapie zu steuern und zu bewirken.

Alternativ oder zusätzlich ist die Steuereinheit in Kombination mit der Stimulationseinheit oder mehreren Stimulationseinheiten dazu ausgebildet, die Abgabe von Stimulationsimpulsen an ein Atrium und einen oder zwei Ventrikeln zu steuern und zu bewirken.

Vorzugsweise ist die Steuereinheit in Kombination mit der Stimulationseinheit oder mehreren Stimulationseinheiten dazu ausgebildet, eine Stimulationstherapie so zu steuern, dass diese den nicht mit der Unterstützungspumpe verbundenen Ventrikel mit der Pumpenfunktion derart synchronisiert, dass eine synchrone Ventrikelfunktion erreicht wird. Insbesondere ist die Steuereinheit ausgebildet, eine adäquate Verzögerung zwischen dem Zeitpunkt der vorgesehenen Abgabe eines rechtsventrikulären Stimulationsimpulses und der Tätigkeit der linksventrikulären Herzunterstützungspumpe zu bewirken.

Gemäß einer bevorzugten Ausführungsvariante sind die Stimulationselektroden ganz oder teilweise integraler Bestandteil der Herzunterstützungspumpe oder eines ihrer Bestandteile wie z.B. Pumpengehäuse, Zuleitungen etc..

Vorzugsweise weist das implantierbare kardiale Therapiegerät einen Impedanzsensor zum Erfassen einer intrakardialen Impedanz und einen Herzstimulator oder wenigstens eine durch die Steuereinheit gesteuerte Stimulationseinheit auf, welche mit der Steuereinheit verbunden sind. Die Steuereinheit kann dann ausgebildet sein, eine Abgabe von Stimulationsimpulsen in Abhängigkeit eines eine intrakardiale Impedanz anzeigen Ausgangssignals des Impedanzsensors zu steuern. Insbesondere kann die Steuereinheit in Verbindung mit dem Impedanzsensor und der Stimulationseinheit dazu ausgebildet sein, die Stimulation auf Basis eines eine kardiale Kontraktilität anzeigenden Ausgangssignals des Impedanzsensors derart zu steuern, dass eine bedarfsgerechte Steigerung der Kontraktilität resultiert. Auf diese Weise ergibt sich ein Kombinationstherapiegerät mit einem CCM-Stimulator (Cardiac Contractility Modulation) zur bedarfsgerechten Steigerung der Kontraktilität. Alternativ oder zusätzlich kann das implantierbare kardiale Therapiegerät eine Defibrillationseinheit aufweisen.

Vorzugsweise weist das implantierbare kardiale Therapiegerät einen Drucksensor als Teil der Herzunterstützungspumpe auf und ist ausgebildet, mittels des Drucksensors einen jeweiligen Mitralklappenschluss zu detektieren und in Abhängigkeit eines jeweils detektierten Mitralklappenschlusses Therapiezeiten zu steuern.

Bezüglich der Steuerung der Stimulationseinheit oder der Stimulationseinheiten bezüglich der Zeitpunkte ggf. abzugebender Stimulationsimpulse kann die Steuereinheit als Kontraktionsablaufkontrolleinheit ausgebildet sein, eine an sich bekannte Closed-Loop-Stimulation (CLS) durchzuführen, auf einer Auswertung der rechtsventrikulären Kontraktionsdynamik basiert, die durch eine oder mehrere intrakardiale Impedanzmessungen mittels eines entsprechenden Impedanzsensors erfasst wird.

Die Steuerung der Abgabe von Stimulationsimpulsen durch die Steuereinheit im Sinne einer Kontraktionsablaufkontrolleinheit kann auch auf einer oder mehrerer Messungen mit einem Hämodynamik-Sensor (HDS) basieren.

Außerdem kann die Steuereinheit als Kontraktionsablaufkontrolleinheit dazu ausgebildet sein, in an sich bekannter Weise automatisch Stimulationserfolgskontrollen durchzuführen. Zusätzlich kann die Steuereinheit basierend auf einer oder mehrerer Stimulationserfolgskontrollen auch zur automatischen Erfassung der Stimulationsschwelle der jeweiligen Herzkammer und zur automatischen Einstellung der jeweiligen Stimulationsimpulsstärke ausgebildet sein.

Gemäß einer besonders bevorzugten Ausführungsvariante ist das implantierbare kardiale Therapiegerät mit einer linksventrikulären Herzunterstützungspumpe und einer Steuereinheit sowie damit verbundenen Sensing- und Stimulationseinheiten einschließlich eines Sensors zur Erfassung der rechtsventrikulären Kontraktiliät bzw. Kontraktionsgeschwindigkeit ausgestattet und dazu ausgebildet, bei Absinken der rechtsventrikulären Kontraktilität das Schlagvolumen mittels der Herzunterstützungspumpe und optional die Stimulationsfrequenz zu senken, um eine rechtsventrikuläre Volumenüberlastung zu vermeiden.

Darüber hinaus ist jede Kombination von mono- und biventrikulären Herzunterstützungspumpen (VAD-Pumpen) mit bekannten Systemen zur kardialen Elektrotherapie denkbar und im Einzelfall nützlich.

Die Erfindung soll nun anhand von Ausführungsbeispielen mit Bezug auf die Figuren näher erläutert werden. Diese zeigen in:
- Figur 1:: ein Herzunterstützungssystem (VAD-System) gemäß dem Stand der Technik;
- Figur 2:: ein Herzunterstützungssystem mit elektrischer Ventrikelsynchronisation;
- Figur 3:: ein Blockschaltbild des Herzunterstützungssystems mit integrierter Resynchronisationseinrichtung; und
- Figur 4:: ein Ablaufdiagramm, dass die Wirkungsweise der Therapiesynchronisationseinheit dargestellt.

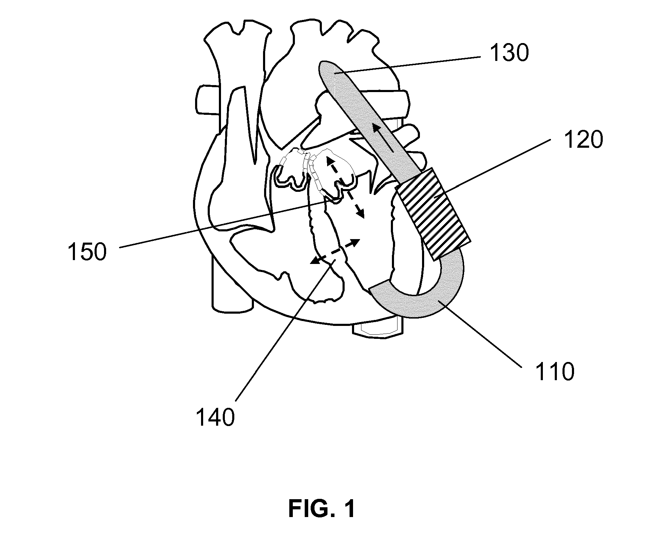

In der Figur 1 ist zunächst der Stand der Technik eines VAD-Systems dargestellt. Zum Anschluss der Unterstützungspumpe (120) wir eine Zuleitung (110) in den linken Ventrikel des Patienten implantiert. Diese Zuleitung hat dabei die Aufgabe, einen Teil des Blutes vom linken Ventrikel zur Pumpe (120) fließen zu lassen. Die Unterstützungspumpe wiederum pumpt das Blutvolumen in die Aorta (130) und entlastet so den linken Ventrikel und damit das myokardiale Sauerstoffdefizit überzukompensieren und ein Reverse-Remodeling zu fördern.

Allerdings sind diese Systeme typischerweise druckgesteuert und verfügen über keine zusätzlichen Methoden zur Resynchronisation der verbleibenden Herzmechanik. Damit bleiben bei den Patienten die für eine ausgeprägte Herzinsuffizienz typischen Störungen der Herzmechanik, die interventrikuläre Dyssynchronie (140) und die atrioventrikuläre Dyssynchronie (150) erhalten. Auf Grund dieser funktionalen Bewegungsstörungen kann angenommen werden, dass eine kurative Therapie mit Reverse Remodeling nennenswert behindert wird.

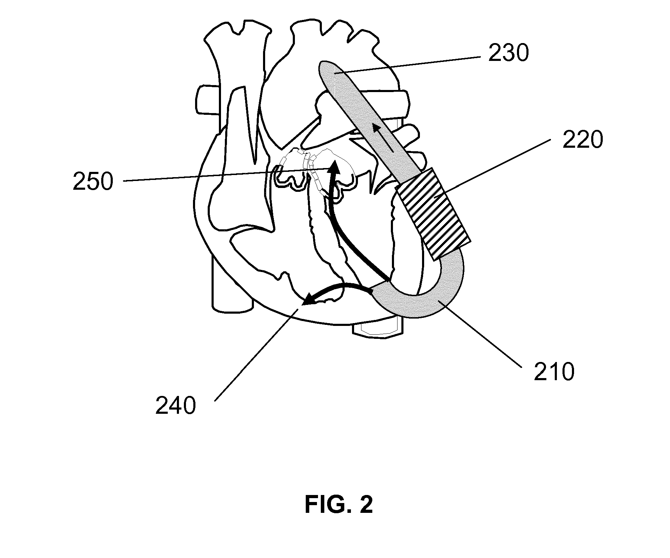

In der Figur 2 ist das erfindungsgemäße System dargestellt. Hier ist die VAD-Pumpe (220) mit ihren Zu- (210) und Abflussleitungen (230) um eine rechtsventrikuläre Elektrodenleitung (240) und eine linksatriale Elektrodenleitung (250) als integrale Bestandteile des VAD-Systems erweitert.

Die linksatriale Elektrodenleitung dient dabei primär der Wahrnehmung einer linksatrialen Erregung, die rechtsventrikuläre Elektrodenleitung der Stimulation des rechten Ventrikels. Die Funktionsweise wird im folgenden Blockschaltbild und Ablaufdiagramm erläutert.

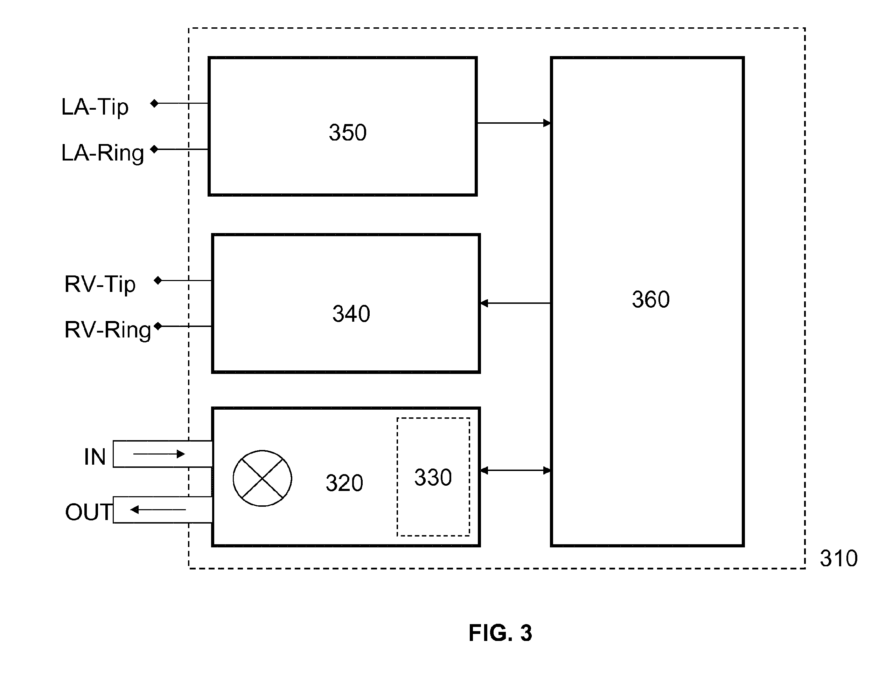

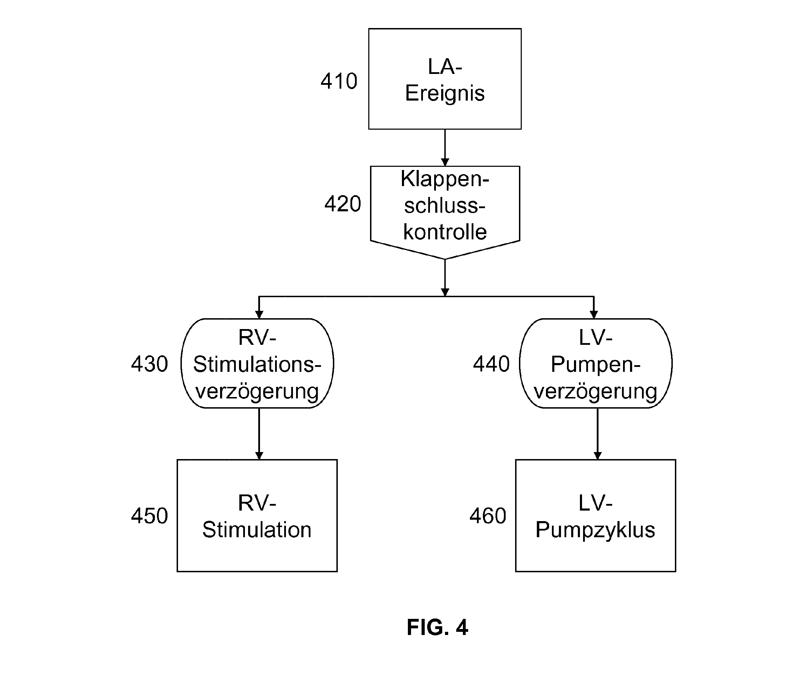

In der Figur 3 ist das Blockschaltbild des VAD-Systems (310) mit integrierter Resynchronisationseinrichtung dargestellt. Dieses umfasst die eigentliche Herzunterstützungspumpe (320) zur Assistenz des linken Ventrikels, die üblicherweise über eine Drucksensorik (330) mit Drucksensor zu deren Steuerung verfügt und mit der linksventrikulären Zu- (IN) und aortalen Abflussleitung (OUT) verbunden ist. Erfindungsgemäß ist dieses System um eine rechtsventrikuläre Stimulations- und Kontraktionsablaufkontrolleinheit (340), verbunden mit einer bipolaren rechtsventrikulären Stimulationselektrode (RV-Tip, RV-Ring) erweitert. Ebenso ist eine linksatriale Wahrnehmungseinheit (350), verbunden mit der ebenfalls bipolaren linksatrialen Wahrnehmungselektrode (LA-Tip, LA-Ring) integriert. Alle genannten Einheiten sind mit einer Therapiesynchronisationseinheit (360) verbunden, deren Wirkungsweise in Figur 4 beschrieben wird.

In der Figur 4 ist die Wirkungsweise der Steuereinheit als Therapiesynchronisationseinheit dargestellt. Eine linksatriale Wahrnehmung (410) führt zunächst zu einer Kontrolle des Mitralklappenschlusses (420) mittels der vorhandenen VAD-Drucksensorik, d.h. der Mitralklappenschluss wird zunächst abgewartet. Anschließend werden zeitgleich zwei unabhängig voneinander programmierbare Verzögerungszeiten (430) und (440) gestartet, um so den für die Resynchronisation optimalen Zeitpunkt für die rechtsventrikuläre Stimulation (450) und linksventrikuläre Pumpenaktivität (460) zu bestimmen. Zusätzlich wird der rechtsventrikuläre Kontraktionsverlauf bzw. die Kontraktionsdynamik erfasst und verwendet, um die programmierbaren Verzögerungszeiten dynamisch an die Resynchronisationsbedürfnisse anzupassen und optional die Herzfrequenz und die LV-Pumpenleistung zu reduzieren, wenn die RV-Kontraktilität dem LV-Schlagvolumen nicht mehr folgen kann. So wird ein Rechtsherzversagen vermieden.

Die Erfindung das Potential, die Therapieeffizienz der VAD-Therapie nennenswert steigern zu können und so ein nachhaltiges Reverse-Remodeling zu fördern, um VAD-Patienten vom VAD-System entwöhnen zu können.

## Patentansprüche

1. Implantierbares kardiales Therapiegerät mit
- einer Herzunterstützungspumpe, die mit einem Ventrikel eines Herzens und einer zugehörigen Arterie zu verbinden und ausgebildet ist, im Einsatzfall Blut aus einem jeweiligen Ventrikel in eine jeweils zugehörige Arterie zu pumpen und so den jeweiligen Ventrikel zu unterstützen/entlasten,
- einer Stimulationseinheit zur elektrischen Stimulation einer Herzkontraktion und
- einer Steuereinheit, die mit der Herzunterstützungspumpe und der Stimulationseinheit steuernd verbunden und ausgebildet ist, die Herzunterstützungspumpe und die Stimulationseinheit derart koordiniert anzusteuern, dass die Stimulationseinheit einen synchronisierten Bewegungsablauf der kardialen Kontraktion bewirkt, während die Herzunterstützungspumpe ventrikelunterstützend arbeitet.

2. Implantierbares kardiales Therapiegerät nach Anspruch 1, **dadurch gekennzeichnet, dass** die Steuereinheit in Verbindung mit der Stimulationseinheit zur koordinierten Abgabe von Stimulationsimpulsen an beide Ventrikel eines Herzens ausgebildet sind.

3. Implantierbares kardiales Therapiegerät nach Anspruch 1 oder 2, **dadurch gekennzeichnet, dass** die Steuereinheit in Kombination mit der Stimulationseinheit oder mehreren Stimulationseinheiten dazu ausgebildet ist, die Abgabe von Stimulationsimpulsen für eine biventrikuläre Stimulationstherapie zu steuern und zu bewirken.

4. Implantierbares kardiales Therapiegerät nach einem der Ansprüche 1 bis 3, **dadurch gekennzeichnet, dass** die Steuereinheit in Kombination mit der Stimulationseinheit oder mehreren Stimulationseinheiten dazu ausgebildet ist, die Abgabe von Stimulationsimpulsen an ein Atrium und einen oder zwei Ventrikeln zu steuern und zu bewirken.

5. Implantierbares kardiales Therapiegerät nach einem der Ansprüche 1 bis 4, **dadurch gekennzeichnet, dass** die Steuereinheit in Kombination mit der Stimulationseinheit oder mehreren Stimulationseinheiten dazu ausgebildet ist, eine Stimulationstherapie so zu steuern, dass diese den nicht mit der Unterstützungspumpe verbundenen Ventrikel mit der Pumpenfunktion derart synchronisiert, dass eine synchrone Ventrikelfunktion erreicht wird.

6. Implantierbares kardiales Therapiegerät nach Anspruch 5, **dadurch gekennzeichnet, dass** die Steuereinheit ausgebildet ist, eine adäquate Verzögerung zwischen dem Zeitpunkt der vorgesehenen Abgabe eines rechtsventrikulären Stimulationsimpulses und der Tätigkeit der linksventrikulären Herzunterstützungspumpe zu bewirken.

7. Implantierbares kardiales Therapiegerät nach einem der Ansprüche 1 bis 6, **dadurch gekennzeichnet, dass** die Stimulationseinheit mit Stimulationselektroden verbunden ist, die ganz oder teilweise integraler Bestandteil der Herzunterstützungspumpe oder eines ihrer Bestandteile sind.

8. Implantierbares kardiales Therapiegerät nach einem der Ansprüche 1 bis 7, **dadurch gekennzeichnet, dass** das implantierbare kardiale Therapiegerät einen Drucksensor als Teil der Herzunterstützungspumpe aufweist und ausgebildet ist, mittels des Drucksensors einen jeweiligen Mitralklappenschluss zu detektieren und in Abhängigkeit eines jeweils detektierten Mitralklappenschlusses Therapiezeiten zu steuern.

9. Implantierbares kardiales Therapiegerät nach einem der Ansprüche 1 bis 8, **dadurch gekennzeichnet, dass** die Steuereinheit als Kontraktionsablaufkontrolleinheit zur Steuerung der Stimulationseinheit oder der Stimulationseinheiten bezüglich der Zeitpunkte ggf. abzugebender Stimulationsimpulse ausgebildet ist, eine Closed-Loop-Stimulation (CLS) durchzuführen, die auf einer Auswertung der rechtsventrikulären Kontraktionsdynamik basiert, welche durch eine oder mehrere intrakardiale Impedanzmessungen mittels eines entsprechenden Impedanzsensors erfasst wird.

10. Implantierbares kardiales Therapiegerät nach einem der Ansprüche 1 bis 9, **dadurch gekennzeichnet, dass** die Steuereinheit als Kontraktionsablaufkontrolleinheit dazu ausgebildet ist, in an sich bekannter Weise automatisch Stimulationserfolgskontrollen durchzuführen.

11. Implantierbares kardiales Therapiegerät nach Anspruch 10, **dadurch gekennzeichnet, dass** die Steuereinheit basierend auf einer oder mehrerer Stimulationserfolgskontrollen zur automatischen Erfassung einer Stimulationsschwelle einer jeweiligen Herzkammer und zur automatischen Einstellung der jeweiligen Stimulationsimpulsstärke ausgebildet sein

12. Implantierbares kardiales Therapiegerät nach einem der Ansprüche 1 bis 10, **dadurch gekennzeichnet, dass** das implantierbare kardiale Therapiegerät mit einer linksventrikulären Herzunterstützungspumpe und mit der Steuereinheit verbundenen Sensing- und Stimulationseinheiten einschließlich und einem mit der Steuereinheit verbundenen Sensor zur Erfassung der rechtsventrikulären Kontraktiliät bzw. Kontraktionsgeschwindigkeit ausgestattet und dazu ausgebildet, bei Absinken der rechtsventrikulären Kontraktilität das Schlagvolumen mittels der Herzunterstützungspumpe und optional die Stimulationsfrequenz zu senken, um eine rechtsventrikuläre Volumenüberlastung zu vermeiden.

13. Implantierbares kardiales Therapiegerät nach einem der Ansprüche 1 bis 12, **dadurch gekennzeichnet, dass** das implantierbare kardiale Therapiegerät einen Impedanzsensor zum erfassen einer intrakardialen Impedanz und einen Herzstimulator oder wenigstens eine durch die Steuereinheit gesteuerte Stimulationseinheit aufweist, welche mit der Steuereinheit verbunden ist, wobei die Steuereinheit ausgebildet ist, eine Abgabe von Stimulationsimpulsen in Abhängigkeit eines eine intrakardiale Impedanz anzeigen Ausgangssignals des Impedanzsensors zu steuern.

14. Implantierbares kardiales Therapiegerät nach Anspruch 13, **dadurch gekennzeichnet, dass** die Steuereinheit in Verbindung mit dem Impedanzsensor und der Stimulationseinheit dazu ausgebildet ist, die Stimulation auf Basis eines eine kardiale Kontraktilität anzeigenden Ausgangssignals des Impedanzsensors derart zu steuern, dass eine bedarfsgerechte Steigerung der Kontraktilität resultiert.

15. Implantierbares kardiales Therapiegerät nach einem der Ansprüche 1 bis 14, **dadurch gekennzeichnet, dass** das implantierbare kardiale Therapiegerät eine Defibrillationseinheit aufweist, die zum Generieren und Abgeben von Defibrillationsimpulsen ausgebildet und zur Steuerung durch die Steuereinheit mit dieser verbunden ist.
